Europäisches Patentamt

European Patent Office

Office européen des brevets

(11) Numéro de publication: **0 237 554 B1**

(12) **FASCICULE DE BREVET EUROPEEN**

(45) Date de publication de fascicule du brevet: **25.03.92**  (51) Int. Cl.5: **A61B 3/10**

(21) Numéro de dépôt: **86905836.2**

(22) Date de dépôt: **19.09.86**

(86) Numéro de dépôt internationale :
**PCT/FR86/00319**

(87) Numéro de publication internationale :
**WO 87/01571 (26.03.87 87/07)**

(54) **PROCEDE ET DISPOSITIF DE MESURE DE LA DIRECTION DU REGARD.**

(30) Priorité: **19.09.85 FR 8513911**

(43) Date de publication de la demande:
**23.09.87 Bulletin 87/39**

(45) Mention de la délivrance du brevet:
**25.03.92 Bulletin 92/13**

(84) Etats contractants désignés:
**DE GB IT SE**

(56) Documents cités:
**EP-A- 0 055 338**
**US-A- 3 533 683**

(73) Titulaire: **INSTITUT NATIONAL DE LA SANTE
ET DE LA RECHERCHE MEDICALE (INSERM)
101, rue de Tolbiac
F-75654 Paris Cédex 13(FR)**

Titulaire: **THOMSON-CSF
51, Esplanade du Général de Gaulle
F-92800 Puteaux(FR)**

(72) Inventeur: **CHARLIER, Jacques
8, Allée Vauban
F-59650 Villeneuve d'Ascq(FR)**
Inventeur: **PARIS, Vincent
25, rue de la Vignette
F-59800 Lille(FR)**
Inventeur: **BARISEAU, Jean-Luc
199, rue Solférino
F-59800 Lille(FR)**

(74) Mandataire: **Benoit, Monique et al
THOMSON-CSF SCPI
F-92045 PARIS LA DEFENSE CEDEX 67(FR)**

EP 0 237 554 B1

## Description

La présente invention concerne la mesure de la direction du regard et elle a plus particulièrement pour objet un procédé et un dispositif de mesure de la direction du regard utilisant comme critère de détection la position relative du reflet cornéen et de la pupille. A titre d'exemple, non exclusif, d'application de l'invention, on peut notamment citer l'examen des fonctions visuelles, l'étude des réflexes pupillomoteurs et oculomoteurs et l'étude de l'oculomotricité, aussi bien dans le domaine de la recherche (ergonomie, neurophysiologie, etc) que de l'industrie (simulateurs d'entraînement par exemple).

On a déjà proposé de nombreux'procédés de mesure de la direction du regard et construit un certain nombre d'appareils permettant de les mettre en oeuvre. L'approche consistant à évaluer la direction du regard à partir de la position relative du reflet cornéen d'une source lumineuse et de la pupille a de nombreux avantages, du fait qu'elle est atraumatique et permet une mesure indépendante de la position de la tête.

On connaît en particulier (FR-A-2 382 056) un procédé de mesure de la direction du regard, suivant lequel on éclaire le globe oculaire par une source de lumière, on recueille l'image de l'oeil contenant l'image de la pupille et celle du reflet de la source sur le dioptre cornéen et on analyse l'image pour déterminer la position du centre de la pupille et celle du centre du reflet pour en déduire la direction du regard par rapport au système de mesure.

En règle générale, on utilise une source infrarouge non visible pour le patient. A condition de placer la source sensiblement sur l'axe optique du système de recueil d'image, la pupille apparaît sous forme d'un disque clair (technique dite de la pupille brillante) et le reflet de la source sur le dioptre sphérique constitué par la face avant de la cornée apparaît comme un point brillant.

On a proposé plusieurs méthodes de traitement du signal vidéo reçu pour déterminer le centre de la pupille et celui du reflet cornéen. La demande de brevet FR-A-2 382 056 utilise la détermination du barycentre des centres de deux segments de pupille de longueur définie. On a également proposé de rechercher le barycentre des centres de plusieurs segments de pupille détectés dans une image (Article de J. CHARLIER et col., New instrument for monitoring eye fixation and pupil size visual field examination. Med. Biol.Eng.Compt.1982, 20, 23-28). On a également proposé la détermination du barycentre de l'ensemble des points de la pupille reconstitué à partir de différents points du contour pupillaire.

Les résultats sont susceptibles d'être très influencés par les variations des niveaux de luminance de l'image en fonction de la taille de la pupille, de la transparence des milieux oculaires et de la réflectance de la rétine du sujet ; des variations des niveaux de luminance de l'image en fonctions de l'ambiance ; des reflets parasites sur la sclérotique ou sur la peau ; des reflets parasites dus à des sources extérieures ou au port de verres correcteurs ; de l'obturation même partielle de l'ouverture pupillaire par les paupières ou les cils.

Des tentatives ont été faites pour limiter ces inconvénients. Elles impliquent des réglages manuels dont la qualité dépend beaucoup de la qualification du personnel qui les effectue et qui impliquent l'intervention de ce personnel. En conséquence ces solutions ne sont satisfaisantes que pour des utilisations en laboratoire. Mais, du fait qu'elles exigent des manipulations relativement complexes pour effectuer des ajustements tenant compte de l'environnement, elles sont très difficilement transposables à des examens effectués par un personnel moins spécialisé et dans des conditions susceptibles de varier dans de larges limites.

L'invention vise à fournir un procédé répondant mieux que ceux antérieurement connus aux exigences de la pratique, notamment en ce qu'il permet d'identifier les bords de la pupille et le reflet cornéen de façon sure même dans des ambiances très variables et en particulier de fournir une discrimination très améliorée, et ce en ne mettant en oeuvre que des moyens de coût modéré.

Dans ce but, l'invention propose notamment un procédé du type ci-dessus défini, caractérisé en ce qu'on détecte les bords de la pupille et du reflet par comparaison de la différence de brillance entre pixels adjacents de la matrice des éléments photosensibles avec plusieurs intervalles de référence prédéterminés.

De façon générale, le procédé permet d'identifier de façon certaine l'image de la pupille et l'image du reflet cornéen. A partir de là, on peut valider les résultats, ce qui facilite la mise en oeuvre, aide à écarter les mesures erronées et n'exige pour la mise en oeuvre que des moyens de coût modéré.

Des raisons d'économie conduiront en règle générale à adopter un capteur de prix modéré, constitué par une caméra à balayage de type télévision, utilisant des éléments sensibles au silicium ou une matrice de capteur à transfert de charge. Dans ce cas, on explore l'image par balayage vidéo (lignes et trame), on détecte les bords sur chaque ligne et on transmet l'es coordonnées des pixels correspondant au bord pour chaque ligne à des moyens de calculs.

Toujours dans un but d'économie, il est souhaitable d'effectuer le traitement numérique à l'aide d'un microprocesseur, qui constitue un bon compromis entre la flexibilité et le coût. Il n'est pas possible, dans la mesure ou l'on veut conserver la fréquence des signaux vidéo standard (supérieure à 6 Mhz pour une résolution spaciale de 256 x 256 points et une révolution temporelle de 50 images par seconde), d'effectuer l'ensemble du traitement à l'aide du microprocesseur. Dans un mode de mise en oeuvre avantageux de l'invention, on effectue dans une première étape la recherche des bords de la pupille et celle du reflet cornéen sur chaque ligne de balayage, on mémorise les résultats obtenus, on identifie la pupille et le reflet cornéen et on en détermine la position au cours d'une seconde étape.

Dans certains cas, l'amélioration de discrimination obtenue du fait que l'on travaille sur des différences d'amplitudes reste insuffisante par suite de l'existence de reflets parasites s'ajoutant à ceux de la source sur les verres correcteurs ou sur la peau. En particulier, la discrimination peut être insuffisante lorsque l'environnement comporte des sources extérieures réfléchies essentiellement par la cornée ou lorsque les mouvements sont tels que la surface sclérale devient perpendiculaire au faisceau provenant de la source d'éclairement.

Pour permettre une meilleure discrimination, on utilise avantageusement dans ce cas plusieurs sources auxiliaires présentant une disposition prédéterminée, fournissant sur la cornée des reflets auxiliaires qui peuvent être détectés et identifiés par reconnaissance de formes. On améliore ainsi l'identification du reflet cornéen. Ces sources auxiliaires sont avantageusement dissociées les unes des autres, pour faciliter l'identification du reflet cornéen. On n'obtiendrait pas les mêmes résultats favorables si l'on utilisait une source linéaire constituant un graphème particulier.

Lorsqu'il est également nécessaire d'améliorer la discrimination des bords de la pupille, l'opération de reconstruction de l'image complète peut être effectuée en prévoyant un critère de sélection supplémentaire, tel que la continuité de la courbure du bord de l'image de la pupille. Grâce à l'application de ce critère de sélection supplémentaire, on identifie les bords de la pupille avec certitude et on peut également déterminer le centre de cette dernière même si l'image ne fournit pas la totalité des bords.

Enfin, la détermination du centre de la pupille peut être réalisé par l'un quelconque des moyens connus. Toutefois, il est particulièrement avantageux d'utiliser un procédé de détermination à partir du tracé mémorisé du bord de la pupille, consistant à rechercher le point pour lequel les variations de distance par rapport aux points successifs de la pupille sont les plus faibles : dans la pratique, ce résultat peut être atteint en utilisant la méthode des moindres carrés. On obtient ainsi une augmentation de la plage de mesure, puisqu'il n'est pas indispensable que la totalité de l'image de la pupille se forme dans le champ du dispositif de visualisation. Grâce à ce processus de reconnaissance de forme, on réduit la sensibilité du procédé à un flou dû par exemple à une erreur de mise au point et on augmente la plage d'utilisation dans les trois dimensions. Enfin, du fait de la capacité accrue d'identification des bords de la pupille et du reflet cornéen, on arrive à les différencier davantage des reflets parasites qui deviennent importants (et interdisent pratiquement la mise en oeuvre de divers procédés connus antérieurement) pour les grands angles d'orientation de l'oeil par rapport au système de mesure, provoquant des reflets parasites sur la sclère.

L'invention propose également un dispositif permettant de mettre le procédé ci-dessus défini, comprenant des moyens d'éclairage du globe oculaire, une caméra à balayage de type télévision destinée à recueillir l'image de la pupille et le reflet de la source, des moyens d'analyse de l'image et de détermination des bords de la pupille et du reflet, caractérisée en ce que lesdits moyens comprennent un circuit destiné à former la différence de brillance de deux pixels adjacents du champ de la caméra, un circuit de comparaison de cette différence à des intervalles prédéterminés. Le dispositif comportera de plus des moyens d'identification des bords de la pupille en fonction des résultats de la comparaison ci-dessus et du reflet. Ces moyens d'identification travailleront avantageusement sur les résultats obtenus sur un nombre de pixels successifs supérieur à 2, généralement inférieur à 10, permettant de détecter la présence du reflet par l'apparition d'une séquence de niveau constant, d'un front montant, puis d'un front descendant et d'un nouveau niveau constant.

De façon similaire, on identifiera les bords de la pupille par la présence d'un front entre deux niveaux constants.

L'invention sera mieux comprise à la lecture de la description qui suit d'un mode particulier d'exécution de l'invention, donnée à titre d'exemple non limitatif. La description se réfère aux dessins qui l'accompagnent, dans lesquels :

- La Figure 1 est un schéma de principe de la partie optique d'un dispositif suivant l'invention,
- la Figure 2 montre une image vidéo de la pupille obtenue à l'aide du dispositif de la Figure 1,
- La Figure 3 est un diagramme de principe montrant les variations d'amplitude du signal vidéo lors du balayage de la ligne indiquée en Figure 2,
- La Figure 4 montre le mode d'élaboration des signaux différentiels et de comparaison avec des intervalles de références, mis en oeuvre dans l'invention,

- La Figure 5 est un synoptique de la partie du dispositif permettant la détection des bords de l'image de la pupille et du reflet cornéen,

Le dispositif qui sera maintenant décrit à titre d'exemple est destiné à déterminer la position instantanée de la direction du regard d'un sujet humain. On considèrera que le dispositif reste, par rapport à la tête, dans un domaine tel que le globe oculaire 10 reste au moins en partie dans le champ. Il peut être considéré comme mobile, par rapport au dispositif, en rotation autour d'un centre 12, lui-même susceptible de se déplacer en translation dans le champ.

Le dispositif comprend une source 14 de lumière infrarouge renvoyée dans le champ de vision, en alignement avec le centre 12, suivant un axe 16 qui sera avantageusement proche de la direction de vision lorsque le globe oculaire est dans sa position médiane, par une lame séparatrice 18 laissant subsister le champ visible pour le sujet et une lame semi-transparente 20. Le faisceau émis par la source 14 (diode électroluminescente par exemple) illumine la face avant du globe oculaire.

Le dispositif qui sera décrit est du type dit à "pupille brillante". Pour cela, on fait coïncider l'axe d'illumination 16 et l'axe optique du système de formation d'images. Le dispositif de formation d'image 22, qu'on supposera être une caméra vidicon à capteurs au silicium ou une caméra à transfert de charges reçoit la lumière par l'intermédiaire de la lame séparatrice 18. Les rayons émis par la source 14 pénètrent alors dans l'oeil, sont réfléchis par la rétine et éclairent la pupille par l'arrière avant de revenir vers la caméra 22 par réflexion sur la lame 18. Il faut cependant noter que l'invention serait tout aussi bien applicable à un montage optique dit à "pupille noire" de constitution générale connue.

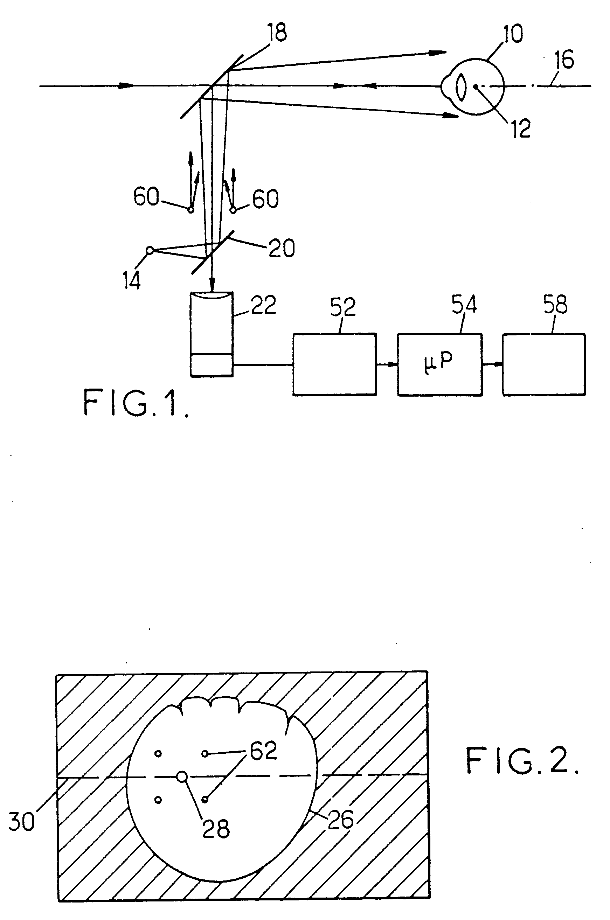

Dans le cas d'un montage optique à "pupille brillante", l'image vidéo fournie par la caméra 22 aura l'allure montrée en Figure 2. Sur le fond sombre apparaît l'image claire de la pupille 26 et, dans cette dernière en général, le reflet cornéen forme une tache brillante 28.

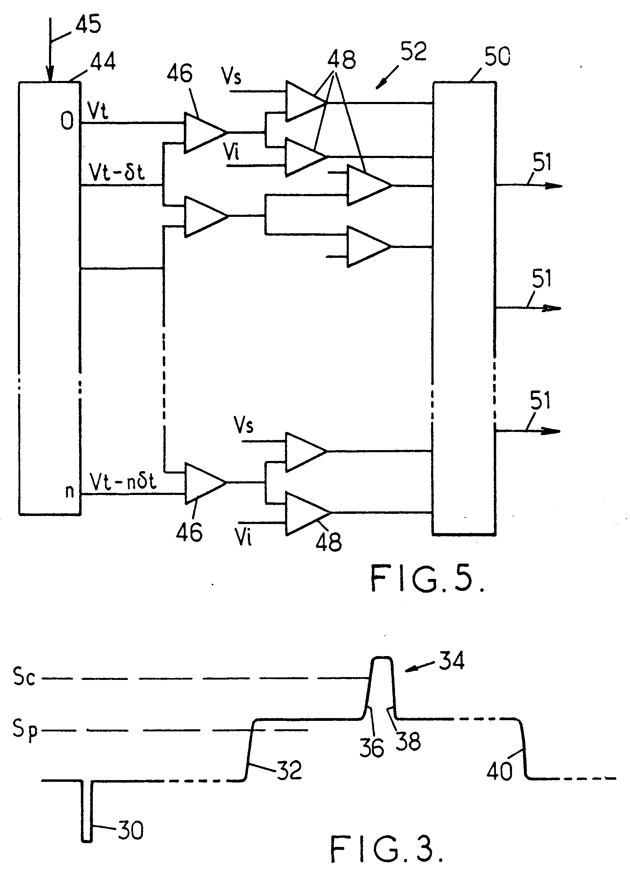

Dans ces conditions, le signal vidéo fourni par la caméra 22 lors d'un balayage d'une ligne 30 traversant la tache 28 aura l'allure montrée en Figure 3. On voit apparaître sur cette figure le signal de synchronisation de ligne 30 puis un front montant 32 correspondant au bord de la pupille, une plage de niveau constant et une séquence 34 d'un front montant 36, d'une plage brève de niveau sensiblement constant puis d'un front descendant 38 correspondant à la tache 28. Enfin, l'autre bord de la pupille apparaît sous forme d'un front descendant 40 entre deux zones de niveau constant.

En principe, il serait possible de détecter les bords de la pupille par le franchissement d'un premier seuil Sp par le signal et ceux de la tache 28 par le franchissement d'un second seuil Sc. Mais dans la pratique cette solution n'est satisfaisante que dans des conditions de laboratoire. L'invention propose de lui substituer l'approche qui sera maintenant décrite.

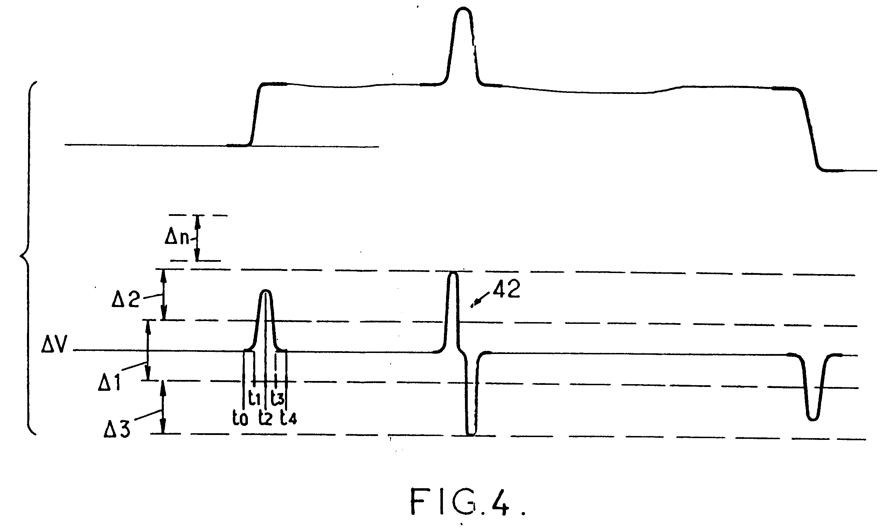

On retrouve à la ligne supérieure de la Figure 4 l'allure générale d'un signal de sortie de la caméra que l'on peut considérer comme représentatif. Conformément à l'invention, on mémorise l'amplitude du signal correspondant à des instants successifs, qui seront généralement séparés par des intervalles de temps constants, et on soustrait deux à deux les signaux successifs, pour obtenir l'amplitude différentielle $\Delta V$ (seconde ligne de la Figure 4).

Cette opération de différenciation élimine l'influence du niveau lumineux de base, sans pour autant introduire de bruit lié à la dérivation électronique. L'opération d'échantillonnage est d'autant plus facile que des caméras comme celles à CCD fournissent directement des signaux représentatifs de pixels successifs, sous forme numérique ou analogique. Il suffit de faire circuler les signaux successifs numérisés dans un registre à décalage pour disposer simultanément d'autant d'échantillons successifs qu'on le désire.

On compare alors les signaux successifs obtenus pour chaque couple d'échantillon avec des intervalles de Valeurs de brillance de référence, tels que les intervalles $\Delta 1$, $\Delta 2$, $\Delta 3$,... $\Delta n$ montrés en Figure 4. Chacun des signaux différentiels peut être affecté à l'un de ces intervalles. A partir du classement ainsi réalisé, un algorithme de détection combinant plusieurs critères de comparaison prédéterminés permettra de déceler les bords de la pupille et le reflet cornéen.

A titre d'exemple, on a montré sur la Figure 4 les signaux différentiels obtenus à des instants successifs t0, t1, t2, t3, t4 correspondant au bord gauche de la pupille. On voit que ce bord est détecté comme une séquence comprenant une plage de signal de niveau constant, un pic montant puis une nouvelle plage de niveau constant. Le front descendant sera détecté comme une séquence comprenant une plage de signal de niveau constant puis un pic descendant et enfin une nouvelle plage de niveau constant. Le niveau cornéen sera de son côté détecté comme une séquence 42 comprenant un niveau constant, un pic montant, un niveau constant bref, puis un pic descendant. L'algorithme de détection, fonctionnant sur un nombre de points qui peut être beaucoup plus important que dans le cas illustré et pouvant n'utiliser que des pixels échantillons à intervalles prédéterminés, permettra d'identifier les bords de la pupille et le reflet cornéen dans la mesure où la tache 28 est traversée par la ligne de balayage. Les parties du signal significatives pour la reconnaissance des formes sont indiquées en traits épais sur la Figure 4.

Plusieurs algorithmes d'identification peuvent être utilisés simultanément. Par ailleurs, les algorithmes utilisés pour les bords de la pupille et le reflet cornéen comprennent des critères de comparaison communs qui pourront être traités aisément par des circuits logiques identiques.

De plus, aux critères de comparaison portant sur les amplitudes différentielles peuvent être associés des critères supplémentaires portant sur les amplitudes absolues, notamment pour obtenir une meilleure discrimination dans la détection du reflet cornéen, caractérisé par un niveau d'amplitude très élevé.

Le procédé qui vient d'être défini peut être mis en oeuvre par un circuit du genre montré, sous forme simplifiée, en Figure 5. Ce circuit comprend une ligne à retard 44 à n position. Chaque échantillon à son tour est appliqué à l'entrée 45 de la ligne à retard. On supposera, dans ce qui suit, que le signal est analogique, ce qui implique l'utilisation d'une ligne à'retard 44 également analogique. Les sorties O à n de la ligne à retard fournissent des signaux représentatifs des échantillons à des instants t, t-ôt, ..., t-nôt. Ces signaux sont soustraits deux à deux par des amplificateurs différentiels 46 et appliqués à des comparateurs à seuil 48. Dans le cas illustré en Figure 5, chaque signal différentiel n'est comparé qu'à un seul intervalle défini par deux valeurs de référence Vi et Vs (qui peuvent correspondre aux limites de l'intervalle $\Delta$ 1 sur la Figure 4). Ainsi, les signaux différentiels seront répartis en trois groupes, suivant qu'aucun des deux comparateurs 48, un seul ou les deux comparateurs fournissent un signal logique de sortie.

Tous les signaux logiques de sortie sont appliqués à un réseau 50 destiné à appliquer les algorithmes de comparaison. Dans la pratique, le réseau 50 peut être constitué par des portes en cascade dont les liaisons sont établies à partir d'un réseau logique programmable ou PLA disponible dans le commerce. Ce réseau comportera des sorties correspondants aux différents échantillons et des signaux logiques seront fournis sur les sorties 51 correspondant au bord de début de la pupille, éventuellement au reflet cornéen, et enfin au bord de fin de la pupille.

Les circuits montrés en Figure 5 constituent avantageusement un interface 52 interposé entre la caméra 22 et un organe de calcul à microprocesseur 54 (Figure 1), muni d'une mémoire vive d'accumulation des résultats fournis par l'interface 52 et un interface 58 de sortie, et éventuellement de rétroaction permettant une régulation de l'éclairement et un réglage d'autofocalisation. La détermination du centre de la pupille et du centre du reflet pourra être effectuée par le calculateur 54 par l'une des approches déjà bien connues, par exemple celle décrite dans le document FR-A-2 382 056) déjà mentionné.

Comme cela a déjà été indiqué plus haut, la discrimination obtenue peut dans certains cas rester insuffisante en ce qui concerne soit le reflet cornéen, soit les bords de la pupille, soit encore les deux.

L'invention propose également des moyens qui ne sont utilisés qu'optionnellement et permettent d'améliorer la discrimination de l'un et/ou de l'autre.

En particulier, pour améliorer la discrimination en ce qui concerne le reflet cornéen, des sources auxiliaires 60 peuvent être prévues autour de l'axe optique 16 et réparties suivant une configuration géométrique déterminée et reconnaissable. Elles peuvent en particulier être réparties sur un carré centré sur l'axe optique. Le faisceau de lumière fourni par ces sources est essentiellement réfléchi par la cornée et donne naissance à des points brillants supplémentaires 62 (Figure 2) qui reproduiront la disposition géométrique des sources (sommets d'un réseau carré dans le cas représenté).

L'image résultante reçue par la caméra 22 comprend alors quatre reflets auxiliaires 62 entourant le reflet central 28. Ces reflets auxiliaires seront détectés de la même façon que le reflet 28 car les sources sont séparées et l'organe de calcul 54 pourra les identifier aisément.

Il faut remarquer au passage que l'image des sources auxiliaires est facilement dissociée des reflets sur la sclérotique, car le rayon de courbure scléral est plus important que celui de la cornée et la distance entre les reflets fournis est très accrue. Par ailleurs, la présence de reflets auxiliaires permet d'améliorer encore la détermination de la position du reflet 28, du fait que l'on peut réaliser une pondération entre les résultats des diverses mesures de localisation.

L'identification de la pupille peut être améliorée également par mise en oeuvre d'un procédé de reconnaissance de forme utilisant le fait que cette image de la pupille présente une forme ovale caractéristique (Figure 2). Le critère de reconnaissance de forme est constitué par l'absence de variation brutale de la courbure. Une approche utilisable pour mettre en oeuvre ce procédé sera maintenant brièvement décrit.

L'ensemble des points détectés comme constituant le début ou la fin de la pupille au cours du balayage par lignes est mémorisé et l'organe de calcul établit une séquence de points dont les distances relatives sont inférieures à un seuil prédéterminé et calcule la longueur de chaque chaîne (définie comme le nombre de points lui appartenant) et la pente locale, entre points successifs.

Les pentes successives sont ensuite comparées entre elles. Pour toute variation de la pente d'un point à un autre, qui est inférieure à un seuil déterminé, on incrémente un compte représentatif de la constance de le courbure d'une unité. Au contraire, pour tout changement de signe ou pour toute variation brutale, on décrémente d'une unité. Toutes les chaînes qui finalement ne correspondent pas à un compte dépassant un seuil prédéterminé sont éliminées. Et les chaînes validées sont utilisées seules pour calculer le centre de la pupille, ainsi que la surface de la pupille lorsqu'on le souhaite.

Ce mode de détermination suppose que l'on utilise le cercle comme modèle de la pupille. Il peut dans d'autres cas être utile d'utiliser l'ellipse ; l'algorithme de détermination du centre sera alors modifié.

Non seulement les méthodes ci-dessus permettent d'identifier la pupille avec davantage de sécurité, mais aussi elles fournissent un critère de qualité de l'image traitée et un critère d'optimisation de l'éclairement des différentes sources de l'appareil, éclairement optimal correspondant à la valeur maximale du critère de qualité. Elles permettent également de fournir un critère aidant à la réalisation d une autofocalisation.

## Revendications

1. Procédé de mesure de la direction du regard, suivant lequel on éclaire le globe oculaire par une source de lumière, on recueille sur une matrice d' éléments photosensibles représentant des pixels l'image de l'oeil contenant l'image de la pupille et celle du reflet de la source sur le dioptre cornéen et on analyse l'image ainsi recueillie pour déterminer la position du centre de la pupille et celle du centre du reflet cornéen pour en déduire la direction du regard par rapport à un système de mesure, caractérisé en ce qu'on détecte les bords de la pupille (26) et du reflet (28) par comparaison de la différence de brillance entre pixels adjacents de la matrice avec plusieurs intervalles de valeurs de brillance de référence prédéterminés.

2. Procédé selon la revendication 1, caractérisé en ce qu'on explore l'image par balayage vidéo par lignes et trame ; on détecte les bords de pupille et le reflet sur chaque ligne et on transmet les coordonnées des pixels correspondant au bord, pour chaque ligne, à des moyens de calcul (52, 54).

3. Procédé selon la revendication 2, caractérisé en ce qu'on effectue dans une première étape la recherche des bords de la pupille et du reflet sur chaque ligne de balayage, on mémorise les résultats obtenus, puis on identifie la pupille et le reflet cornéen et on détermine la position de la pupille au cours d'une seconde étape.

4. Procédé selon la revendication 1, 2 ou 3, caractérisé en ce qu'on utilise plusieurs sources auxiliaires (60) présentant une disposition prédéterminée, fournissant sur la cornée des reflets auxiliaires qu'on détecte et identifie par reconnaissance de formes.

5. Procédé suivant la revendication 4, caractérisé en ce que les sources auxiliaires sont dissociées entre elles.

6. Procédé selon la revendication 2, caractérisé en ce qu'on identifie, sur chaque ligne les bords de la pupille par la présence, sur une ligne, d'une séquence constituée par une brillance constante, puis une différence représentant un front montant ou descendant, et de nouveau, une brillance constante.

7. Procédé selon la revendication 3, caractérisé en ce que l'on identifie le centre de la pupille à partir des bords ou d'une portion des bords de cette dernière par recherche du point dont la distance aux divers points du bord présente un minimum de variation.

8. Procédé selon la revendication 3, caractérisé en ce que l'on reconnaît le bord de la pupille par l'absence de variation brutale de la courbure dudit bord.

9. Procédé selon la revendication 8, caractérisé en ce qu'on mémorise l'ensemble des points détectés comme constituant le début ou la fin de la pupille au cours du balayage par lignes, on calcule la distance relative des points, on compare ces distances relatives à un seuil prédéterminé, on sélectionne les séquences de points dont les distances relatives sont inférieures au seuil, on calcule la longueur de chaque chaîne, définie comme le nombre de points lui appartenant, et la pente locale entre points successifs et on compare les pentes successives entre elles.

10. Dispositif permettant de mettre en oeuvre le procédé suivant la revendication 1, comprenant une source (14) d'éclairage du globe oculaire, une caméra à balayage de type télévision (22) destinée à recueillir l'image de la pupille et du reflet de la source à l'aide d'une matrice d' éléments photosensibles représentant des pixels, et des moyens (52) d'analyse de l'image et de détermination des bords de la pupille et du reflet, caractérisé en ce que lesdits moyens d'analyse comprennent un circuit (46) destiné à former la différence de brillance de deux pixels successifs de la matrice de la caméra, un circuit (48) de comparaison de cette différence avec des intervalles de Brillance prédéterminés et des moyens (50) d'identification des bords de la pupille et du reflet en fonction des résultats de la comparaison ci-dessus.

11. Dispositif selon la revendication 10, caractérisé en ce que les pixels successifs comparés sont prélevés à intervalles déterminés dans les pixels d'une ligne de la matrice.

12. Dispositif selon la revendication 10, caractérisé en ce que la présence du reflet est détectée par l'apparition d'une séquence de brillance de niveau constant, d'un front montant puis d'un front descendant et d'un nouveau niveau constant.

13. Dispositif selon l'une quelconque des revendications 10 à 12, caractérisé en ce que les moyens d'analyse (52) constituent un interface fournissant un signal représentatif de la position des bords de la pupille et des bords du reflet à un calculateur (54) pour détermination du centre de la pupille et du centre du reflet.

14. Dispositif selon la revendication 13, caractérisé en ce qu'il comprend également des sources auxiliaires (60) présentant une disposition prédéterminée autour de la source d'éclairement et en ce que le calculateur (54) est prévu pour identifier ladite disposition dans l'image.

**Claims**

1. A method of measurement of the direction of observation, in accordance with which the eyeball is illuminated by a source of light, the image of the illuminated eye is projected on a matrix of photosensitive elements representing pixels, containing the image of the pupil and that of the reflection of the source on the corneal optical interface and the image projected in this manner is analyzed in order to ascertain the position of the center of the pupil and that of the center of the cornea reflection in order to deduce therefrom the direction of observation in relation to a system of measurement, characterized in that the edges of the pupil (26) and the refection (28) are detected by comparison of the difference in brilliance between adjacent pixels of the matrix with a plurality of brilliance value intervals with a predetermined reference.

2. The method as claimed in claim 1, characterized in that the image is explored by video line and frame sweeping; the edges of the pupil and the reflection on each line and the coordinates of pixels corresponding to the edge are transmitted, for each line, to calculating means (52 and 54).

3. The method as claimed in claim 2, characterized in that in a first stage the edges of the pupil and of the reflection are searched for on each sweep line, the results obtained are stored, then the pupil and the reflection are identified and the position of the pupil is ascertained during the course of a second stage.

4. The method as claimed in any one of the preceding claims 1, 2 and 3, characterized in that a plurality of auxiliary sources (60) are utilized having a predetermined disposition and supplying the cornea with additional reflections, which are detected and identified by recognizing the forms thereof.

5. The method as claimed in claim 4, characterized in that the auxiliary sources are mutually dissociated.

6. The method as claimed in claim 2, characterized in that on each line the edges of the pupil are identified on a line by the presence of a sequence constituted by a constant brilliance and then a difference representative of an ascending or descending front and again a constant brilliance.

7. The method as claimed in claim 3, characterized in that the center of the pupil is identified on the basis of the edges or of a part of the edges of the same by searching for the point whose distance from various points of the edge presents a minimum of variation.

8. The method as claimed in claim 3, characterized in that the edge of the pupil is detected by the absence of abrupt variation in the curvature of the said edge.

9. The method as claimed in claim 8, characterized in that the totality of the detected points is stored as constituting the start or the end of the pupil during the course of sweeping on lines, the relative distance of the points is calculated, these relative distances are compared with a predetermined threshold, the sequences of the points are selected whose relative distances are below the threshold, the length each chain is calculated which is defined as the number of points belonging to it, and the local slopes between successive points and the successive points are compared with each other.

10. A device for performing the method as claimed in claim 1, comprising a source (14) for the illumination of the eyeball, a television-type sweep camera (22) adapted to receive the image of the pupil and of the reflection of the source with the aid of a matrix of photosensitive elements representing pixels, and means (52) for the analysis of the image and the ascertainment of the edges of the pupil and of the reflection, characterized in that the said analysis means comprise a circuit (46) adapted to derive the difference in brilliance of two successive pixels of the matrix of the camera, a circuit (48) for the comparison of the said difference with predetermined intervals in brilliance and means (50) for the identification of the edges of the pupil and of the reflection as a function of the results of the comparison above.

11. The device as claimed in claim 10, characterized in that the successive compared pixels are sampled at predetermined intervals in the pixels of a line of the) matrix.

12. The device as claimed in claim 10, characterized in that the presence of the reflection is detected by the appearance of a constant level brilliance sequence, of a front which ascends and then of a front which descends and of a constant new level.

13. The device as claimed in any one of the preceding claims 10 through 12, characterized in that the analysis means (52) constitute an interface supplying a signal representative of the position of the edges of the pupil and of the edges of the reflection to a calculating means (54) in order to ascertain the center of the pupil and of the center of the reflection.

14. The device as claimed in claim 13, characterized in that it comprises furthermore auxiliary sources (60) having a predetermined disposition around the source of illumination and in that the calculating means (54) is provided in order to identify the said disposition in the image.

**Patentansprüche**

1. Verfahren zum Messen der Blickrichtung, gemäß dem der Augapfel mit einer Lichtquelle beleuchtet wird, das Bild des beleuchteten Auges, das das Bild der Pupille und dasjenige des Reflexes der Quelle auf dem Hornhautdiopter enthält, auf einer Matrix von lichtempfindlichen Elementen, die Bildpunkte darstellen, aufgefangen wird und das so aufgefangene Bild analysiert wird, um die Position des Zentrums der Pupille und diejenige des Zentrums des Hornhautreflexes zu bestimmen, um daraus die Blickrichtung in bezug auf ein Meßsystem abzuleiten, dadurch gekennzeichnet, daß die Ränder der Pupille (26) und des Reflexes (28) durch den Vergleich der Leuchtkraftdifferenz zwischen benachbarten Bildpunkten der Matrix mit einer Mehrzahl von vorgegebenen Intervallen von Bezugsleuchtkraftwerten ermittelt werden.

2. Verfahren gemäß Anspruch 1, dadurch gekennzeichnet, daß das Bild durch Zeilen- und Teilbild-Videoabtastung untersucht wird, die Ränder der Pupille und des Reflexes in jeder Zeile erfaßt werden und die Koordinaten der dem Rand entsprechenden Bildpunkte für jede Zeile an Rechenmittel (52, 54) übertragen werden.

3. Verfahren gemäß Anspruch 2, dadurch gekennzeichnet, daß in einem ersten Schritt die Ränder der Pupille und des Reflexes in jeder Abtastzeile gesucht und die erhaltenen Ergebnisse gespeichert werden und dann die Pupille und der Hornhautreflex identifiziert werden und im Verlauf eines zweiten Schrittes die Position der Pupille bestimmt wird.

4. Verfahren gemäß Anspruch 1, 2 oder 3, dadurch gekennzeichnet, daß eine Mehrzahl von Hilfsquellen (60) verwendet werden, die eine vorgegebene Anordnung besitzen und auf der Hornhaut Hilfsreflexe hervorrufen, die durch Formwiedererkennung erfaßt und identifiziert werden.

5. Verfahren gemäß Anspruch 4, dadurch gekennzeichnet, daß die Hilfsquellen voneinander getrennt sind.

6. Verfahren gemäß Anspruch 2, dadurch gekennzeichnet, daß in jeder Zeile die Ränder der Pupille durch eine in einer Zeile vorhandene Abfolge, die von einer konstanten Leuchtkraft, dann von einer eine steigende oder eine fallende Flanke darstellenden Differenz und dann erneut von einer konstanten Leuchtkraft gebildet wird, identifiziert werden.

7. Verfahren gemäß Anspruch 3, dadurch gekennzeichnet, daß das Zentrum der Pupille anhand der Ränder oder eines Teils der Ränder der letzteren identifiziert wird, indem der Punkt gesucht wird, dessen Abstand von verschiedenen Randpunkten eine minimale Variation aufweist.

8. Verfahren gemäß Anspruch 3, dadurch gekennzeichnet, daß der Rand der Pupille durch das Nichtvorliegen einer starken Variation der Randkrümmung wiedererkannt wird.

9. Verfahren gemäß Anspruch 8, dadurch gekennzeichnet, daß die Gesamtheit der im Verlauf der Zeilenabtastung erfaßten Punkte, die den Anfang oder das Ende der Pupille bilden, gespeichert wird, der relative Abstand der Punkte berechnet wird, diese relativen Abstände mit einem vorgegebenen Schwellenwert verglichen werden, die Punktfolgen, deren relative Abstände kleiner als der Schwellenwert sind, ausgewählt werden, die Länge einer jeden Kette, die durch die Anzahl der ihr zugehörigen Punkte definiert wird, und die lokale Steigung zwischen aufeinanderfolgenden Punkten berechnet werden und die aufeinanderfolgenden Steigungen untereinander verglichen werden.

10. Einrichtung zur Ausführung des Verfahrens gemäß Anspruch 1, mit einer Quelle (14) für die Beleuchtung des Augapfels, einer Abtastkamera vom Typ einer Fernsehkamera (22), die dazu vorgesehen ist, das Bild der Pupille und des Reflexes der Quelle mit Hilfe einer Matrix von Bildpunkte darstellenden lichtempfindlichen Elementen einzufangen, und Mitteln (52) für die Analyse des Bildes und die Bestimmung der Ränder der Pupille und des Reflexes, dadurch gekennzeichnet, daß die Analysemittel eine Schaltung (46), die dazu vorgesehen ist, die Leuchtkraftdifferenz zwischen zwei aufeinanderfolgenden Bildpunkten der Matrix der Kamera zu bilden, eine Schaltung (48) für den Vergleich dieser Differenz mit vorgegebenen Leuchtkraftintervallen und Mittel (50) für die Identifizierung der Ränder der Pupille und des Reflexes in Abhängigkeit vom obigen Vergleich umfassen.

11. Einrichtung gemäß Anspruch 10, dadurch gekennzeichnet, daß die verglichenen, aufeinanderfolgenden Bildpunkte den Bildpunkten einer Zeile der Matrix in bestimmten Intervallen entnommen werden.

12. Einrichtung gemäß Anspruch 10, dadurch gekennzeichnet, daß das Vorliegen des Reflexes durch das Auftreten einer Leuchtkraftabfolge mit konstantem Niveau, einer steigenden Flanke, dann einer fallenden Flanke und einem erneuten konstanten Niveau ermittelt wird.

13. Einrichtung gemäß einem der Ansprüche 10 bis 12, dadurch gekennzeichnet, daß die Analysemittel (52) eine Schnittstelle bilden, die ein die Position der Ränder der Pupille und der Ränder des Reflexes darstellendes Signal an eine Recheneinrichtung (54) liefert, um das Zentrum der Pupille und das Zentrum des Reflexes zu bestimmen.

14. Einrichtung gemäß Anspruch 13, dadurch gekennzeichnet, daß sie außerdem Hilfsquellen (60) umfaßt, die eine vorgegebene Anordnung um die Beleuchtungsquelle aufweisen, und daß die Recheneinrichtung (54) dazu vorgesehen ist, diese Anordnung im Bild zu identifizieren.

FIG.1.

FIG.2.

FIG.5.

FIG.3.

FIG.4.